# EUROPEAN PATENT APPLICATION

(11) **EP 4 506 448 A1**
(43) Date of publication of application: **12.02.2025**
(21) Application number: 23190589.4
(22) Date of filing: 09.08.2023
(51) Int. Cl.: C12N 5/078, C12N 5/0789

(54) **GENERATION OF IMMORTALIZED PROGENITOR MEGAKARYOCYTES FROM HUMAN HEMATOPOIETIC STEM CELLS FOR LARGE SCALE PLATELET PRODUCTION INTO MICROFLUIDICS DEVICE**

(71) Applicant: HemostOD SA, 1025 Saint-Sulpice (CH)
(72) Inventor: BURNIER, Laurent, 1006 Lausanne (CH); HUMBERT, Magali, 1680 Romont (CH); ROCCA, Céline, 1024 Ecublens VD (CH)
(74) Representative: DTS Patent- und Rechtsanwälte PartmbB

(57) **Abstract**

The invention relates to a method for producing immortalized megakaryocytes (102) and/or immortalized megakaryocyte progenitors from CD34+ HSPCs (100), the method comprising: introducing an expression cassette comprising at least one oncogene into the CD34+ HSPCs (100) by transduction, in particular lentiviral (LV) transduction,
wherein the transduced CD34+ HSPCs (100) are configured to express the at least one oncogene, at least upon induction of expression by an activator,
wherein the transduced CD34+ HSPCs (100) are immortalized through expression of the a least one oncogene.

Further, the present invention relates to a method for producing a platelet (104), a platelet (104) and a platelet (104) for use in a method for the treatment of thrombocytopenia, bleeding or for use in regenerative medicine.

## Description

The present invention relates to a method for producing immortalized megakaryocytes and/or an immortalized megakaryocytic cell line, a method for producing a platelet and a platelet for use in a method for the treatment and/or prevention of several disease conditions or regenerative medicine.

Platelet infusion in patients with very low level of platelets, as it is the case in thrombocytopenia, is often the only therapeutic option to avoid life threatening bleedings in patients with hypoproliferative bone marrow. Besides thrombocytopenia, platelet infusion in patients can also be used as therapy in other disease conditions, such as e.g. leukemia, bone marrow transplantation, anticancer treatment, etc.

Platelets can be isolated during blood donation, but their short shelf life of about 5 to 7 days negatively impacts their availability, especially during health crisis such as epidemics. Moreover, platelets from donors always contain human plasma, red blood cells and residual leukocytes, leading to adverse events after transfusion, unless the concentrate is treated with devices such as filters or pathogen-reduction techniques which decrease the risk of adverse events.

The currently accepted theory is that platelets are formed and released into the bloodstream by precursor cells called megakaryocytes (MKs) derived from pluripotent hematopoietic and progenitor stem cells (HSPCs) that reside within the bone marrow (BM) (Wang et al.).

Megakaryocytopoiesis is the process by which megakaryocytes and ultimately platelets develop. The differentiation of platelets from HSCs proceeds by multiple different cell lineages which involve many genes encoding several transcription factors and proteins.

Hematopoietic stem and progenitor cells differentiate into Multipotential Progenitors, which differentiate into Common Myeloid Progenitor, which differentiate into Megakaryocyte-erythroid progenitors, which differentiate into megakaryocytes. Immature megakaryocytes mature into mature megakaryocytes. Platelets are produced after cytoplasmatic maturation of megakaryocytes and proplatelet formation.

Large scale production of platelets for therapeutic use is a challenge, mainly for the following reasons: First, *In vitro,* MKs do not produce as many platelets as *in vivo,* despite some advances to generate more platelets. Second, *in vitro* expansion of MKs or of their progenitors is limited by biology. In addition, the source of MKs or their progenitors is limited since they are either isolated from peripheral blood (PB) or BM aspiration from donors.

Even though large-scale donation would be achievable, correct isolation, maturation and platelet production would be too expensive and would lead to unsolvable variation in platelet production and quality, impacting safety and efficacy, preventing industrialization for therapeutic use.

Identification of new techniques to generate functional platelets ex *vivo* (*in vitro*) would allow to meet clinical demands, assist in reducing the cost of safety testing, enhance transfusion medicine, and support patient treatment. If one could use a megakaryocytic cell line as single source for platelet production, it would therefore be possible to produce an indefinite number of platelets of equivalent quality.

Over the years, a lot of efforts have been dedicated to the ex *vivo* production of platelets. The sources of MKs and platelets can be grouped into four different categories: (1) hematopoietic stem and progenitor cells (HSPCs) (2), cell reprogramming (3), non-hematopoietic sources and (4) artificial platelets.

To overcome the limited self-renewal capacity of HSPCs, scientists have developed human induced pluripotent stem cells (iPSCs) that can differentiate towards MKs, given the adequate growth conditions (including but not limited to a cocktail of cytokines), able to produce platelet-like particles (Chen, Sugimoto and Eto, 2023). They provide the advantages of being an inexhaustible self-renewable source of MKs, can be genetically modified to become universal donor platelets (Figueiredo *et al.,* 2010), and can be grown under serum-free conditions (Pick *et al.,* 2013); however, their clinical benefit has not been proven yet.
Other types of cells have also been exploited to produce MKs and platelets. Endothelial cells as well as fibroblasts can transdifferentiate towards MKs under specific conditions (Ono *et al.,* 2012; Lis *et al.,* 2017). However, the most promising cell type are adipose tissue-derived stromal cells, in particular because of its availability (Tozawa *et al.,* 2019).

Finally, scientists are also trying to develop artificial platelets utilizing biosynthetic particle constructs that functionally emulate various haemostatic mechanisms of platelets. There are several avenues to develop these artificial platelets: protein-decorated artificial platelet systems, peptide-decorated artificial platelet systems and also hetero-multivalent decorations (Luc *et al.,* 2022). Despite artificial platelets seem to be an excellent alternative solution, particularly for its universal aspect, a lot of studies need to be performed to assess thrombotic risks, scalability, cost, and possible immune responses in case of multiple infusions.

EP2708597B1 discloses a method of producing polyploidized MKs. The method comprises the step of obtaining the megakaryocytes by forcing expression of an oncogene selected from the MYC family of genes, and BMI1, in cells at any differentiation stage from hematopoietic progenitor cells to MKs before polyploidization, forcing expression of BCL-XL in MKs before polyploidization, and culturing and proliferating the resulting cells. Further disclosed is a method of producing a platelet from the produced polyploidized MKs.

Although described as functional, these MKs and platelets are not originated from their natural progenitors, the HSPCs. Thus, they may be lacking important features, such as number of platelets produced per MK, and hemostatic potency.

It is an object of the present invention to provide a (further) method for producing immortalized megakaryocytes and/or an immortalized megakaryocytic cell line, in particular able for providing functional platelets, in particular functional clinical-grade platelets, from natural progenitors.

The object is solved according to the present invention by a method, with the features of claim 1, accordingly, a method for producing immortalized megakaryocytes and/or immortalized megakaryocyte progenitors from CD34+ HSPCs, the method comprising:
introducing an expression cassette comprising at least one oncogene into the CD34+ HSPCs by transduction, in particular lentiviral transduction,
wherein the transduced CD34+ HSPCs are configured to express the at least one oncogene, at least upon induction of expression by an activator,
wherein the transduced CD34+ HSPCs are immortalized through expression of the a least one oncogene.

The invention is based on the basic idea that CD34+ HSPCs, the original source of stem and progenitor cells from which MKs and platelets originate, are immortalized by transduction of at least one oncogene, in particular at least one exogeneous oncogene. The transduced CD34+ HSPCs being immortalized and capable of differentiating into immortalized MKs, in particular in the presence of specific cytokine cocktails, resulting in an immortalized megakaryocytic cell line with almost an infinite proliferative capacity, while keeping MK differentiation properties.

Platelets provided by MKs obtained by the above method are as similar as possible to the ones circulating *in vivo.* An essentially unlimited cell source for *in vitro* production of clinically relevant platelets is provided. Almost infinite proliferating capacity will allow an unlimited stock of MKs without the need to generate new cell lines. This aspect permits increasing homogeneity among the batches of platelets produced. Overall, the method allows to produce rapidly enough platelets for an infusion, obtain platelets functionally similar to the ones circulating in vivo, obtain universality and reduce cost of production.

An immortalized cell line can be defined a population of from a which would normally not proliferate indefinitely but, due to mutation, have evaded normal cellular senescence and instead can keep undergoing division. The cells can therefore be grown for prolonged periods *in vitro.* The mutations required for immortality can occur naturally or be intentionally induced for experimental purposes.

CD34 is a transmembrane phosphoglycoprotein protein encoded by the CD34 gene in humans, mice, rats and other species. The CD34 protein is a member of a family of single-pass transmembrane sialomucin proteins that show expression on early haematopoietic and vascular-associated progenitor cells.

Lentiviruses can be used to transduce cells with an expression cassette. The cassette contains a constitutive (for instance EF1a) or an inducible, for instance tetracycline-dependent (for instance TRE) promoter. Next to the promoter, at least one coding sequence encoding for at least one oncogene can be found. In a preferred embodiment, third generation lentiviruses are used for transduction. Transduction using lentivirus enables the integration of the expression cassette within the genome of the cells avoiding thus the expression loss of the oncogene.

In particular, the produced immortalized megakaryocytes and/or immortalized megakaryocyte progenitors can be capable of providing functional platelets upon maturation, in particular functional clinical-grade platelets. As described previously, an unlimited cell source for *in vitro* production platelet production of clinically relevant platelets is provided, which allows to produce rapidly enough platelets for an infusion, obtain platelets functionally similar to the ones circulating in vivo, obtain universality and reduce cost of production. The differentiated immortalized megakaryocytes and/or immortalized megakaryocyte progenitors can be mechanically manipulated to generate platelets on demand. Infusion of generated platelets can then be given intravenously to people with low platelet count or poor function to stop (acute treatment) or prevent bleeding (prophylactic treatment).

Immortalized MKs and/or an immortalized MK cell line derived from HSPCs, in particular CD34+ HSPCs, overcome the various challenges described above. An immortalized MK cell line derived from HSPCs, in particular CD34+ HSPCs, has the advantage to grow for a longer period as compared to MK progenitor cells derived from HSPCs, in particular CD34+ HSPCs, and in a minimal growth media. Differentiated immortalized MKs provide the possibility to freeze and thaw mature MKs as needed, representing an infinite source of MKs to produce platelets at a minimal cost.

The at least one oncogene can be E6, E7, JAK2, JAK2 V617F, YAP, MLL-AF9, CDK4 R24C, CCND1, HOXB8, GATA1s, FLI1, ERG, BCK2L1, hTERT or MEIS1.

E6 and E7 are the major oncogenes of the human papillomavirus (HPV). E6 inhibits p53 and causes loss in cell cycle regulation. E7 is involved in pRb mediated deregulation of the cell cycle.

Expression of mutant JAK2 has been shown to increase the proportion of megakaryocyte-biased CD41high hematopoietic stem cells. In particular a subset of hematopoietic stem cells exists that are defined by elevated expression of CD41 and show bias for differentiation towards megakaryocytes.

Yes-associated protein 1 (YAP1) is a protein that acts as a transcription coregulator that promotes transcription of genes involved in cellular proliferation and suppressing apoptotic genes.

MLL-AF9: MLL= Mixed-lineage leukemia 1 (now renamed lysine K-specific methyl transferase 2A or KMT2A). AF-9 is a protein encoded by the MLLT3 gene. Fusion protein resulting from the chromosomal translocation t(9;11)(p22;q23). MLL-AF9 is a fusion protein associated with aggressive leukemias of both the myeloid and lymphoid lineage and may therefore be used to immortalized HSPC in vitro.

Cyclin-dependent kinase 4 (mutated R24C) (CDK4 R24C): The mutation R24C renders the CDK4 protein insensitive to inhibition by INK4 proteins including p16INK4a. It can facilitate tumorigenesis and escape from cellular senescence.

Cyclin D1 (CCND1): This cyclin forms a complex with and functions as a regulatory subunit of CDK4, whose activity is required for cell cycle G1/S transition.

Homeobox B8 (HOXB8): HoxB8 overexpression can immortalise hematopoietic progenitor cells in a growth-factor-dependant manner and cooperates with interleukin-3 to cause myeloid leukaemia.

Short form of the GATA1 transcription factor (GATA1s): GATA1s is a naturally occurring isoform of GATA1 that lacks the N-terminal transactivation domain and is crucial for maintaining the proliferative status of megakaryocytic, erythroid and eosinophil precursors.

Friend leukaemia integration 1 transcription factor (FLI1): FLI1 is necessary for the lineage diversification process of the Megakaryocyte-Erythroid Progenitor (MEP). It has been shown that its overexpression can downregulate expression of erythroid-specific genes and up-regulate the expression of megakaryocyte-specific genes.

ETS-Related Gene (ERG): ERG is necessary for the lineage diversification process of the Megakaryocyte-Erythroid Progenitor (MEP). It has been shown that its overexpression can downregulate expression of erythroid-specific genes and up-regulate the expression of megakaryocyte-specific genes.

B-cell lymphoma 2 like 1 (BCL2L1): BCL2L1 is a potent inhibitor of cell-death.

Human telomerase reverse transcriptase (hTERT): hTERT can elongate the telomeres, which as a consequence, can increase the lifespan of cells by allowing for indefinite division without shortening of telomeres.

Myeloid ecotropic viral integration site-1 (MEIS1): overexpression of MEIS1 can inhibit myeloid differentiation and enhance erythroid and megakaryocytic development.

In a preferred embodiment, the activator is a tetracycline or estrogen-receptor modulator, wherein the method further comprises inducing expression of the at least one oncogene by the activator. The activator can be Tetracycline or a derivate thereof. For instance, the activator can be Chlortetracycline, Oxytetracycline, Demectocycline, Lymecycline, Meclocycline, Methacycline, Minocycline, Rolitetetracycline, Doxycycline, Tiigecyline, Ervacycline, Sarecycline, Omadacycline. The estrogen-receptor modulator can be a Selective estrogen receptor modulators. In particular, the estrogen-receptor modulator can be Tamoxifene, Toremifene, Reloxifene, Ospemifene, Anordrin, Bazedoxifene, Broparestrol, Clomifene, Cyclofenil, Lasofoxifene or Ormeloxifene. Thus, the expression of the oncogene can be activated (alternative wording: induced) by the addition of the respective activator (e.g. tetracycline-controlled transcriptional activation. The advantage of such an inducible system is that the oncogene is not expressed all the time, but the time point or time span of expression of the oncogene can be determined precisely.

In a preferred embodiment, the method further comprises culturing the transduced CD34+ HSPCs, wherein culturing the transduced CD34+ HSPCs comprises expanding the transduced CD34+ HSPC in the presence of a first set of cytokines and/or the activator. In other words, the presence of a specific set of cytokines and/or the activator, allows and/or favours the expansion of the transduced CD34+ HSPC. The set of cytokines can be understood as a cocktail of cytokines, with an effect determined by combination of the single cytokines and their concentration in the cocktail.

In general, the set of cytokines can comprise at least one of Thrombopoietin (TPO), TPO receptor agonist, stem cell factor (SCF), interleukin 3 (IL-3), interleukin 6 (IL-6), interleukin 9 (IL-9) and interleukin 11 (IL-11). In particular, these cytokines, alone or in combination are advantageous for CD34+ HSPC expansion.

In a further preferred embodiment, the method further comprises differentiating the transduced and/or expanded CD34+ HSPCs into immortalized megakaryocytes and/or immortalized megakaryocyte progenitors, in the presence of a second set of cytokines. In particular, the second set of cytokines allows and/or enables and/or induces the differentiation into immortalized megakaryocytes and/or immortalized megakaryocyte progenitors.

The second set of cytokines can differ from the first set of cytokines, in particular in the number of cytokines, the type of cytokines, and/or the concentration of at least one cytokine. As the effect of a set of cytokines is determined by the combination (i.e. composition) of the single cytokines and their concentration in the cocktail, the second set of cytokines, differing in the number of cytokines, the type of cytokines, and/or the concentration of at least one cytokines, may have a completely different effect than the first set of cytokines. Overall, this enables that different cell culture steps (e.g. expansion, differentiation and/or induction) are induced and/or enabled by different sets of cytokines.

In a preferred embodiment, the set of cytokines comprises: TPO or TPO receptor agonist (low concentration), SCF and II-3 for cell expansion and TPO (higher concentration) and SCF for the differentiation into mature MK.
The method can further comprise the step of culturing the immortalized megakaryocytes and/or immortalized megakaryocyte progenitors, wherein the culturing optionally comprises the differentiation into mature megakaryocytes. Culturing, in general, allows keeping a cell line for a period of time.

In a preferred embodiment the step of culturing the CD34+ HSPCs, immortalized megakaryocytes and/or the immortalized megakaryocytic cell line and/or the mature megakaryocytes is conducted in the absence of a serum and/or in the absence of a feeder cell. Serum is quite expensive, thus, culturing the CD34+ HSPCs, immortalized megakaryocytes and/or the immortalized megakaryocytic cell line and/or the mature megakaryocyte in serum-free conditions saves production costs. Feeder cells support the growth of cells in culture by contributing an undefined and complex mixture of extracellular matrix (ECM) components, and growth factors. Culturing cells supported by feeder cells is expensive and complex. Thus, culturing the CD34+ HSPCs, immortalized megakaryocytes and/or the immortalized megakaryocytic cell line and/or the mature megakaryocyte without feeder cells saves production costs.

In a further preferred embodiment, the CD34+ HSPCs are derived from peripheral blood (PB), bone marrow (BM) or cord blood (CB) of a single donor, in particular a single human donor. A single donor is advantageous, as production time and costs can be minimized because no pool of donors is required (e.g. only one health test, only one blood collection, etc. is required).

Procedures to differentiate HSPCs into MKs are nowadays well-known and several sources of CD34+ HSPCs are available: cord-blood (CB), bone marrow (BM) and peripheral blood (PB). Basically, the CD34+ HSPCs used for the production of MKs according to the invention can be derived from CB, BM and/or PB.

However, CD34+ HSPCs from CB, BM or PB may be not equally available and not giving the same quality of platelets derived from MKs. CB represents an abundant source of immature HSPCs and has been extensively used to produce MKs. Several scientific groups showed that CB -CD34+ HSPC derived MKs differ from BM- and PB-MKs, and this can affect the functionality of the resulting platelets (Miyazaki *et al.,* 2000; Gaur *et al.,* 2006).

Adult BM may represent a very interesting source of HSPCs because CD34+ HSPCs are more frequent in BM than in PB, but the invasive procedure to obtain it limits it access.

Finally, PB may be a very adequate solution. Blood donations are quite good accessible and although quantity of HSPCs is lower in PB, they can be mobilized in the blood donor using G-CSF and/or Plerixafor. CD34+ HSPCs derived from PB give rise to functional MKs and platelets upon differentiation in vitro (Salunkhe, Papadopoulos and Gutiérrez, 2015). In all cases, independently of the source, HSPCs (i.e. not immortalized HSPCs) have limited proliferation properties and are not suitable for providing platelets on demand on the long term. Culture conditions of the cells have two purposes: expansion/survival of the cells but also the differentiation of the HSPC and the immortalized MK cell line into MK. This needs to be done in chemically defined conditions to prevent compromising the final product.

In one embodiment, the step of culturing the CD34+ HSPCs, immortalized megakaryocytes and/or the immortalized megakaryocytic cell line is conducted for a duration of about one day to at least 3 months.

In a further embodiment, the CD34+ HSPCs are derived from peripheral blood, bone marrow or core blood of multiple donors.

In a further embodiment, the step of culturing the CD34+ HSPCs, immortalized megakaryocytes and/or immortalized is conducted at 37°C.

Another important aspect when producing platelets may be the universality of the product. Indeed, allogenic platelet refractoriness may often be caused by incompatibility of human leukocyte antigens (HLA) and antibodies in the recipient binding donor epitopes.

Universal platelets could be generated mainly through gene modification techniques targeting Beta-2-Microglobulin (B2M), which encodes the β2m-light chain component of HLA class I antigens. B2M-knockdown through RNA interference technology reduces HLA class I expression, whereas B2M-knockout via endonucleases (ex: CRISPR/Cas9) creates HLA-negative cells. It has been showed that cultured HLA-silenced or ablated megakaryocytes and platelets are functional while evading immune detection (Suzuki et al., 2020). In the experimental procedure, while transducing the CD34+ HSPC to immortalize them, addition of a lentiviral vector encoding for a miRNA B2M can be considered. This may allow to generate a source of universal platelets able to escape their destruction through natural killer cells, avoiding then an immune response that could be detrimental in patients in need of platelets transfusion.

A further important aspect may be the capacity to freeze MKs. The set of transgenes and choice of promoters describe above may increase MK survival after freezing and thawing, allowing to generate a bank of mature MK.

Further, it is an object of the present invention to provide a method for the production of platelets, while the produced platelets have similar functionalities to the ones circulating in vivo, obtain universality, while the costs of the production are low.

The object is solved according to the present invention by a method, with the features of claim 12, accordingly, a method for
producing a platelet, comprising:
obtaining immortalized megakaryocytes and/or immortalized megakaryocyte progenitors by using the method according to any one of claims 1 to 11 and culturing the immortalized megakaryocytes and/or an immortalized megakaryocyte progenitor,
differentiating the immortalized megakaryocytes and/or cells of the immortalized megakaryocyte progenitors into mature megakaryocytes,
producing platelets by mechanically manipulating the mature megakaryocytes, optionally in a bioreactor or microfluidic device.

In particular, platelets can be generated from mature MKs. Platelet production itself can be done by persons skilled in the art. Hence the invention describes more precisely a method to produce engineered megakaryocytes capable of producing engineered platelets.

The invention also relates to a platelet produced by the method according to claim 12.

The invention further related to a platelet produced by the method according to claim 12 for use in a method for the treatment of thrombocytopenia, bleeding or for use in regenerative medicine. In other words, a platelet produced by the method according to the invention can be used in a therapeutic approach.

It can be seen as an advantage of the invention that CD34+ HSPCs are the original (natural) source of MKs and platelets. Compared to other techniques using different cell sources, the megakaryocytes and/or platelets produced by the method(s) according to the invention are physiologically closer to the ones found in vivo. During the immortalization process, CD34+ HSPCs and MKs could be modified to generate universal platelets. Altogether, the approach of the present invention could practically and economically become a solution to support the needs in platelets in transfusion units, therapeutic and/or preventive applications and/or regenerative medicine. Further details and advantages of the present invention shall now be disclosed in the connection with the drawings.

It is shown in
Fig. 1 a schema of a method for producing immortalized megakaryocytes and/or an immortalized megakaryocytic cell line from CD34+ HSPC;
Fig. 2 a schema of a method of producing a platelet;
Fig. 3 an exemplary illustration of a method for producing immortalized megakaryocytes and/or an immortalized megakaryocytic cell line from CD34+ HSPCs, and an exemplary illustration of a method for producing a platelet; and
Fig. 4 results of an experiment of transducing CD34+ HSPCs with lentiviral vectors encoding for the proteins BCL2L1, HOXB8 and rtTA.

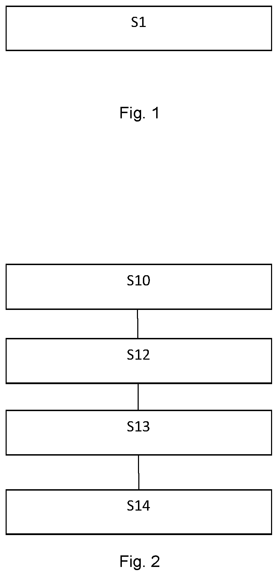

**Fig. 1** shows a schema of a method for producing immortalized megakaryocytes and/or an immortalized megakaryocytic cell line from CD34+ HSPCs 100, according to the present invention.

In a step S1 an expression cassette comprising at least one oncogene is introduced into the CD34+ HSPCs 100 by transduction, in particular lentiviral LV transduction.

The transduced CD34+ HSPCs 100 express the at least one oncogene, at least upon induction of expression by an activator.

The transduced CD34+ HSPCs 100 are immortalized through expression of the a least one oncogene.

The produced immortalized megakaryocytes 102 and/or immortalized megakaryocyte progenitors are/is capable of providing functional platelets 104 upon maturation, in particular functional clinical-grade platelets 104.

The at least one oncogene is E6, E7, JAK2, JAK2 V617F, YAP, MLL-AF9, CDK4 R24C, CCND1, HOXB8, GATA1s, FLI1, ERG, BCK2L1, hTERT or MEIS1.

The activator is a tetracycline or estrogen-receptor modulator, wherein the method further comprises inducing expression of the at least one oncogene by the activator.

Not shown in the present embodiment is that the method could further comprise a step S2 of culturing the transduced CD34+ HSPCs 100.

Culturing the transduced CD34+ HSPCs 100 comprises expanding the transduced CD34+ HSPCs 100 in the presence of a first set of cytokines and/or the activator.

The set of cytokines comprises at least one of TPO, TPO receptor agonist, SCF, IL-3, IL-6, IL-9 and IL-11.

Not shown in this embodiment is that the method can further comprise a step S3 of differentiating the transduced and/or expanded CD34+ HSPCs 100 into immortalized megakaryocytes 102 and/or immortalized megakaryocyte progenitors, in the presence of a second set of cytokines.

The second set of cytokines can differ from the first set of cytokines, in particular in the number of cytokines, the type of cytokines, and/or the concentration of at least one cytokine.

Not shown in this embodiment is that the method can further comprise a step S5 of culturing the immortalized megakaryocytes 102 and/or immortalized megakaryocyte progenitors.

Optionally, the culturing comprises the differentiation into mature megakaryocytes 102.

Optionally, the step of culturing the CD34+ HSPCs 100, immortalized megakaryocytes 102 and/or the immortalized megakaryocytic cell line and/or the mature megakaryocytes 102 is conducted in the absence of a serum and/or in the absence of a feeder cell.

Not explicitly shown in this embodiment is that the CD34+ HSPCs 100 are derived from peripheral blood, bone marrow or core blood of a single donor.

Not explicitly shown is that the single donor is a single human donor.

**Fig. 2** shows a schema of a method of producing a platelet 104.

In a step S10, immortalized megakaryocytes 102 and/or immortalized megakaryocyte progenitors are obtained by the method according to Fig. 1.

In step 12, the immortalized megakaryocytes 102 and/or immortalized megakaryocyte progenitors are cultured.

In step S13, the immortalized megakaryocytes 102 and/or cells of the immortalized megakaryocyte progenitors are differentiated into mature megakaryocytes 102.

In step S14, platelets 104 are produced by mechanically manipulating the mature megakaryocytes 102.

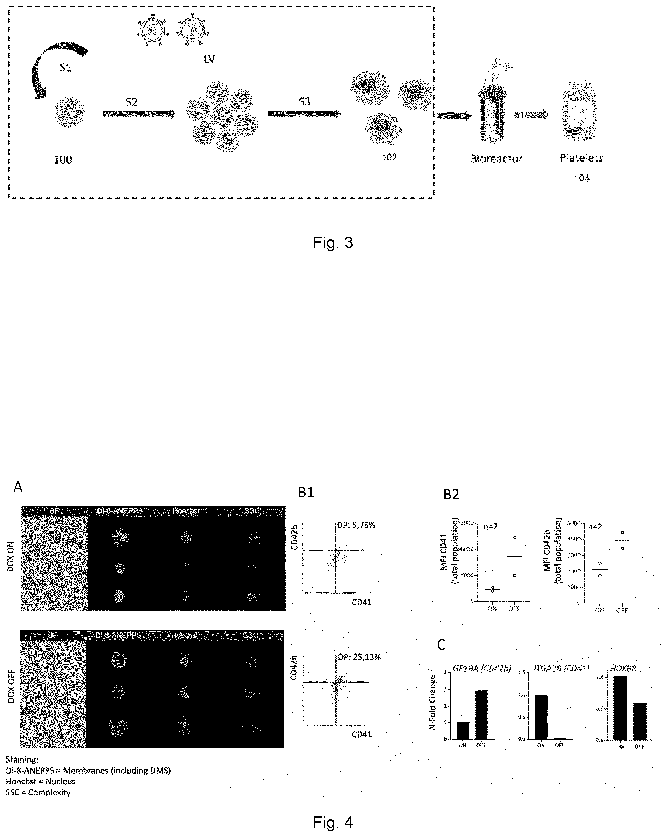

Optionally, step S14 is conducted in a bioreactor or microfluidic device, cf. Fig. 3.

Not shown in Fig. 2 is that a platelet 104 produced by the method can be used in a method for the treatment of thrombocytopenia, bleeding or for use in regenerative medicine.

**Fig. 3** shows an exemplary illustration of a method for producing immortalized megakaryocytes 102 and/or an immortalized megakaryocytic cell line from CD34+ HSPCs 100, according to the present invention (area bounded by the dashed line) and an exemplary method for producing a platelet 104.

The principle of the methods shown in Fig. 3 is shown in Figs. 1 and 2, respectively.

The illustration of the method for producing immortalized megakaryocytes 102 and/or an immortalized megakaryocytic cell line from CD34+ HSPCs 100, according to the present invention is shown in the area bounded by the dashed line.

The illustration of the method of producing a platelet 104 is shown by Fig. 3 (complete).

Starting material are human CD34+ HPSC 100 from peripheral blood or bone marrow of a single donor.

All blood cell lineages are produced via functional maturation of a rare population of multipotent HPSCs that can proliferate by self-renewal and differentiation. HSPC can be found in several organs, such as peripheral blood, bone marrow, and umbilical cord blood.

A first lentivirus LV is used to transduce cells with a first expression cassette.

A second lentivirus LV is used to transduce cells with a second expression cassette.

In this embodiment, the first expression cassette comprises a constitutive promoter (EF1a).

In this embodiment, the second expression cassette comprises a tetracycline-dependent promoter (TRE).

Not shown in this embodiment is that a lentivirus LV could comprise more than one expression cassette or an expression cassette with a constitutive and an inducible promoter.

Next to a promoter, there can be one or multiple coding sequences encoding for oncogenes (E6, E7, JAK2, JAK2 V617F, YAP, MLL-AF9, CDK4 R24C, CCND1, HOXB8, GATA1s, FLI1, ERG, BCK2L1, hTERT or MEIS1).

Transduction using lentivirus LV allows the integration of the expression cassette within the genome of the transduced cells avoiding thus the expression loss of the oncogene.

Culture conditions of the cells have two purposes: expansion/survival of the cells but also the differentiation of the HSPC 100 and the immortalized MK cell line into megakaryocytes 102.

This needs to be done in chemically defined conditions to prevent compromising the final product.

Cytokines that are used in this embodiment are TPO or TPO receptor agonist (at low concentration), SCF and II-3 for the expansion of HSPCs 100 and TPO (higher concentration than used for expansion) and SCF for the differentiation into mature MK 102.

Not explicitly shown is that in this embodiment a specific combination of oncogenes to immortalize CD34+ HSPCs 100 is used, i.e. HOXB8 together with BCL2L1.

HOXB8 protein is a sequence-specific transcription factor, with ability to arrest myeloid differentiation and allow the progenitors to divide indefinitely while BCL2L1 protein is a critical anti-apoptotic protein.

In this experimental setting, BCL2L1 is under the control of the EF1a promoter and is constitutively expressed while HOXB8 is under the control of the TER promoter which depends on the co-transduction of reverse tetracycline-controlled transactivator (rtTA) (LV-EF1a-rtTa) and the presence of the activator doxycycline to be expressed.

For the production of platelets 104, the immortalized megakaryocytes 102 are differentiated into mature megakaryocytes 102.

Platelets 104 are produced by mechanically manipulating the mature megakaryocytes 102, here by means of a bioreactor.

**Fig. 4** shows results of an experiment of transducing CD34+ HSPCs with lentiviral vectors encoding for the proteins BCL2L1, HOXB8 and rtTA, (cf. Fig. 3, bounded by the dashed line).

After transduction of CD34+ HSPCs 100 and expansion for several weeks (here 12 weeks) in presence of the activator doxycycline, the cells have been kept in culture for an extra week in differentiation media in absence of doxycycline.

During the time of culture, the co-respective untransduced cells (control cells, cultured under the same conditions) did not survive (data not shown), suggesting an immortalization of the cells transduced with the oncogenes only.

Results show that in differentiation media without doxycycline, cells acquire megakaryocytic features as suggested by expression of the CD41 and CD42b markers (seen by flow cytometry for both makers (Figs. B1, B2) but only for CD42b for qPCR (Fig. 4C). Moreover, microscopy analyses show acquisition of pluri-ploidy for some of the cells which is another characteristic of megakaryocytes (Fig. 4A).

It should be noted that several oncogenes and/or combinations of oncogenes have been tested to immortalize CD34+ cells. The example shown here is just one combination providing encouraging data regarding immortalization of CD34+ HSPCs 100 and differentiation into megakaryocytes 102.

### References

- 100: Hematopoietic stem and progenitor cell, HSPC
- 102: Megakaryocyte, MK
- 104: Platelet(s)

- LV: lentivirus

- S1: method step
- S2: method step
- S3: method step
- S12: method step
- S14: method step
- S15: method step

### Bibliography

Chen, S.J., Sugimoto, N. and Eto, K. (2023) 'Ex vivo manufacturing of platelets: beyond the first-in-human clinical trial using autologous iPSC-platelets', International Journal of Hematology, 117(3), pp. 349-355. Available at: https://doi.org/10.1007/s12185-022-03512-8.
Di Buduo, C.A. et al. (2015) 'Programmable 3D silk bone marrow niche for platelet generation ex vivo and modeling of megakaryopoiesis pathologies', Blood, 125(14), pp. 2254-2264. Available at: https://doi.org/10.1182/blood-2014-08-595561.
Figueiredo, C. et al. (2010) 'Generation of HLA-deficient platelets from hematopoietic progenitor cells: GENERATION OF HLA-DEFICIENT PLTs', Transfusion, 50(8), pp. 1690-1701. Available at: https://doi.org/10.1111/j.1537-2995.2010.02644.x.
Gaur, M. et al. (2006) 'Megakaryocytes derived from human embryonic stem cells: a genetically tractable system to study megakaryocytopoiesis and integrin function', Journal of thrombosis and haemostasis: JTH, 4(2), pp. 436-442. Available at: https://doi.org/10.1111/j.1538-7836.2006.01744.x.
Lis, R. et al. (2017) 'Conversion of adult endothelium to immunocompetent haematopoietic stem cells', Nature, 545(7655), pp. 439-445. Available at: https://doi.org/10.1038/nature22326.
Luc, N.F. et al. (2022) 'Bioinspired artificial platelets: past, present and future', Platelets, 33(1), pp. 35-47. Available at: https://doi.org/10.1080/09537104.2021.1967916.
Miyazaki, R. et al. (2000) 'Comparative analyses of megakaryocytes derived from cord blood and bone marrow', British Journal of Haematology, 108(3), pp. 602-609. Available at: https://doi.org/10.1046/j.1365-2141.2000.01854.x.
Moreau, T. et al. (2016) `Large-scale production of megakaryocytes from human pluripotent stem cells by chemically defined forward programming', Nature Communications, 7(1), p. 11208. Available at: https://doi.org/10.1038/ncomms11208.
Ono, Y. et al. (2012) `Induction of functional platelets from mouse and human fibroblasts by p45NF-E2/Maf', Blood, 120(18), pp. 3812-3821. Available at: https://doi.org/10.1182/blood-2012-02-413617.
Pick, M. et al. (2013) 'Generation of Megakaryocytic Progenitors from Human Embryonic Stem Cells in a Feeder- and Serum-Free Medium', PLoS ONE. Edited by E.T. Zambidis, 8(2), p. e55530. Available at: https://doi.org/10.1371/journal.pone.0055530.
Salunkhe, V., Papadopoulos, P. and Gutiérrez, L. (2015) 'Culture of Megakaryocytes from Human Peripheral Blood Mononuclear Cells', BIO-PROTOCOL, 5(21). Available at: https://doi.org/10.21769/BioProtoc.1639.
Strassel, C. et al. (2016) 'Aryl hydrocarbon receptor-dependent enrichment of a megakaryocytic precursor with a high potential to produce proplatelets', Blood, 127(18), pp. 2231-2240. Available at: https://doi.org/10.1182/blood-2015-09-670208.
Suzuki, D. et al. (2020) `iPSC-Derived Platelets Depleted of HLA Class I Are Inert to Anti-HLA Class I and Natural Killer Cell Immunity', Stem Cell Reports, 14(1), pp. 49-59. Available at: https://doi.org/10.1016/j.stemcr.2019.11.011.
Tozawa, K. et al. (2019) 'Megakaryocytes and platelets from a novel human adipose tissue-derived mesenchymal stem cell line', Blood, 133(7), pp. 633-643. Available at: https://doi.org/10.1182/blood-2018-04-842641.
Wang, B. et al. (2016) `Platelet generation in vivo and in vitro', Springerplus, 5(1): 787.

## Claims

1. A method for producing immortalized megakaryocytes (102) and/or immortalized megakaryocyte progenitors from CD34+ HSPCs (100), the method comprising:
introducing an expression cassette comprising at least one oncogene into the CD34+ HSPCs (100) by transduction, in particular lentiviral (LV) transduction,
wherein the transduced CD34+ HSPCs (100) are configured to express the at least one oncogene, at least upon induction of expression by an activator,
wherein the transduced CD34+ HSPCs (100) are immortalized through expression of the a least one oncogene.

2. The method according to claim 1, **characterized in that** the produced immortalized megakaryocytes (102) and/or immortalized megakaryocyte progenitors are/is capable of providing functional platelets (104) upon maturation, in particular functional clinical-grade platelets (104).

3. The method according to claim 1 or claim 2, **characterized in that** the at least one oncogene is E6, E7, JAK2, JAK2 V617F, YAP, MLL-AF9, CDK4 R24C, CCND1, HOXB8, GATA1s, FLI1, ERG, BCK2L1, hTERT or MEIS1.

4. The method according to one of the preceding claims, **characterized in that** the activator is a tetracycline or estrogen-receptor modulator, wherein the method further comprises inducing expression of the at least one oncogene by the activator.

5. The method according to one of the preceding claims, further comprising culturing the transduced CD34+ HSPCs (100),
wherein culturing the transduced CD34+ HSPCs (100) comprises expanding the transduced CD34+ HSPCs (100) in the presence of a first set of cytokines and/or the activator.

6. The method according to claim 5, **characterized in that** the set of cytokines comprises at least one of TPO, TPO receptor agonist, SCF, IL-3, IL-6, IL-9 and IL-11.

7. The method according to one of the preceding claims, further comprising differentiating the transduced and/or expanded CD34+ HSPCs (100) into immortalized megakaryocytes (102) and/or immortalized megakaryocyte progenitors, in the presence of a second set of cytokines.

8. The method according to claim 7, **characterized in that** the second set of cytokines differs from the first set of cytokines, in particular in the number of cytokines, the type of cytokines, and/or the concentration of at least one cytokine.

9. The method according to claim 7 or claim 8, further comprising the step of culturing the immortalized megakaryocytes (102) and/or immortalized megakaryocyte progenitors, wherein the culturing optionally comprises the differentiation into mature megakaryocytes (102).

10. The method according to one of claims 5 to 9, **characterized in that** the step of culturing the CD34+ HSPCs (100), immortalized megakaryocytes (102) and/or the immortalized megakaryocytic cell line and/or the mature megakaryocytes (102) is conducted in the absence of a serum and/or in the absence of a feeder cell.

11. The method according to one of the preceding claims, **characterized in that** the CD34+ HSPCs (100) are derived from peripheral blood, bone marrow or core blood of a single donor, in particular a single human donor.

12. A method of producing a platelet (104), comprising:
obtaining immortalized megakaryocytes (102) and/or immortalized megakaryocyte progenitors by using the method according to any one of claims 1 to 11 and culturing the immortalized megakaryocytes (102) and/or immortalized megakaryocyte progenitors,
differentiating the immortalized megakaryocytes (102) and/or cells of the immortalized megakaryocyte progenitors into mature megakaryocytes (102),
producing platelets (104) by mechanically manipulating the mature megakaryocytes (102), optionally in a bioreactor or microfluidic device.

13. A platelet (104) produced by the method according to claim 12.

14. A platelet (104) produced by the method according to claim 12 for use in a method for the treatment of thrombocytopenia, bleeding or for use in regenerative medicine.
